# EUROPEAN PATENT APPLICATION

(11) **EP 4 636 773 A1**
(43) Date of publication of application: **22.10.2025**
(21) Application number: 23903672.6
(22) Date of filing: 01.09.2023
(51) Int. Cl.: G16B 40/00, G16B 25/20, G16B 50/00, G16B 45/00

(54) **METHOD FOR GENERATING COMPONENT FOR ASSAY, AND DEVICE FOR GENERATING COMPONENT FOR ASSAY PERFORMING SAME**

(30) Priority: 12.12.2022 KR 20220172460
(71) Applicant: Seegene, Inc., Seoul 05548 (KR)
(72) Inventor: SONG, Chang-Hee, Seoul 05548 (KR); JOO, Weon-Woo, Seoul 05548 (KR); LEE, Hyeong-Sub, Seoul 05548 (KR); KOO, Jae-Kwan, Seoul 05548 (KR)
(74) Representative: Viering, Jentschura & Partner mbB Patent- und Rechtsanwälte
(86) International application number: PCT/KR2023/013113
(87) International publication number: WO 2024/128468

(57) **Abstract**

A method for generating a component for an assay, the method comprising: acquiring molecular diagnostic amplification data generated by using a molecular diagnostic assay for a plurality of target nucleic acid molecules; and generating the component for the assay based at least partially on the molecular diagnostic amplification data, wherein the component for the assay includes: a processing/analysis module including sub-modules for processing and analyzing, in the target presence/absence determining software, the molecular diagnostic amplification data for the plurality of target nucleic acid molecules; and a parameter value referenced by the sub-modules, and wherein a sub-module predefined to be called for the processing and analyzing the molecular diagnostic amplification data among the sub-modules processes and analyzes the molecular diagnostic amplification data for the plurality of target nucleic acid molecules by referencing the parameter value in response to a request of the target presence/absence determining software.

## Description

### [Technical Field]

The disclosure relates to a method and device for generating an assay component for a molecular diagnostic assay of a plurality of target nucleic acid molecules.

### [Background Art]

Currently, molecular diagnostics is a rapidly growing field in the in vitro diagnostics market for early diagnosis of diseases. Among the molecular diagnostic methods, methods using nucleic acids are particularly useful for diagnosing causal genetic factors caused by infections by viruses, bacteria, etc., based on their high specificity and sensitivity.

Most diagnostic methods using nucleic acids use nucleic acid amplification reactions that amplify target nucleic acids (e.g., viral or bacterial nucleic acids). As a representative example, a polymerase chain-reaction (PCR) among the nucleic acid amplification reactions includes a repeated cycle process of denaturation of doublestranded DNA, annealing of an oligonucleotide primer to a DNA template, and primer extension by DNA polymerase (Mullis et al., U.S. Patent Nos. 4,683,195, 4,683,202, and 4,800,159; Saiki et al., Science 230:1350-1354)1985)). Other methods for amplifying nucleic acids may include a ligase chain reaction (LCR) (U.S. Pat. Nos. 4,683,195 and 4,683,202; PCR Protocols: A Guide to Methods and Applications (Innis et al., eds., 1990)), strand displacement amplification (SDA) (Walker, et al. Nucleic Acids Res. 20(7):1691-6 (1992); Walker PCR Methods Appl 3(1):1-6 (1993)), transcription-mediated amplification (Phyffer, et al., J. Clin. Microbiol. 34:834-841 (1996); Vuorinen, et al., J. Clin. Microbiol. 33:1856-1859 (1995)), nucleic acid sequence-based amplification (NASBA) (Compton, Nature 350(6313):91-2 (1991)), rolling circle amplification (RCA) (Lisby, Mol. Biotechnol. 12(1):75-99 (1999); Hatchet et al., Genet. Anal. 15(2):35-40 (1999)) and Q-beta replicase (Lizardi et al., BiolTechnology 6:1197(1988)), loop-mediated isothermal amplification (LAMP, Y. Mori, H. Kanda and T. Notomi, J. Infect. Chemother., 2013, 19, 404-411), recombinase polymerase amplification (RPA, J. Li, J. Macdonald and F. von Stetten, Analyst, 2018, 144, 31-67), etc.

Recently, multiplex diagnostic technologies are being used to detect the plurality of target nucleic acids within a single tube based on the nucleic acid amplification reactions. For example, examples of the nucleic acid amplification reactions include various multiplex technologies for detecting several types of viruses at once using the PCR and LAMP methods, etc., described above.

In these nucleic acid amplification reaction technologies, an amplification for target nucleic acids of interest is performed, and a determining process for detecting presence of target nucleic acid molecules is performed using a data set acquired during the amplification. The data set acquired during the amplification is acquired from equipment performing an amplification. One or more determining modules for determining the presence/absence of the target nucleic acid molecules may be applied to the data set. Looking into these determining modules, the types and execution order of functions performed may vary depending on the characteristics of the target nucleic acid molecules, or additional functions may be performed or some functions may not be performed. In addition, the types of parameters to be referenced in these determining modules may vary depending on the characteristics of the target nucleic acid molecules, or even if the types of parameters are the same, the parameter values may differ.

In addition, the data set acquired during the amplification is used in the determining process for detecting the presence of the target. Here, the above-described determining process may be performed in software in some cases.

The software may apply the data set acquired during the amplification for the target nucleic acid molecules to the determining module, thereby identifying whether there is the target.

The determining module refers to the parameter value in the process of identifying whether there are the target nucleic acid molecules by applying the data set. Conventionally, the software has been developed in a method that the determining module and the parameter value referenced by the determining module are hard-coded. The hard-coding is a method of directly embedding data into a source code when implementing the software.

However, in order to add a new parameter or module to be detected by the software, or change an operation order of the determining module that is previously used for target analysis, there was the inconvenience of having to individually reflect the changes in the software, implement a new version of software, and then deploy the new version of software.

### [Disclosure]

### [Technical Problem]

A problem to be solved according to an embodiment is to generate different an assay components for each molecular diagnostic assay.

Another problem to be solved according to an embodiment is to efficiently deploy and manage a deployed processing/analysis module and a parameter value through an assay component separated from target presence/absence determining software.

However, the problem to be solved by the present disclosure is not limited to that mentioned above, and other problems to be solved that are not mentioned may be clearly understood by those of ordinary skill in the art to which the present disclosure belongs from the following description.

### [Technical Solution]

In accordance with an embodiment of a method for generating an assay component performed by a device for generating an assay component used in target presence/absence determining software that displays molecular diagnostic amplification data, the method comprising: acquiring molecular diagnostic amplification data generated by using a molecular diagnostic assay for a plurality of target nucleic acid molecules; and generating the assay component based at least partially on the molecular diagnostic amplification data, wherein the assay component includes: a processing/analysis module including sub-modules for processing and analyzing, in the target presence/absence determining software, the molecular diagnostic amplification data for the plurality of target nucleic acid molecules; and a parameter value referenced by the sub-modules, and wherein a sub-module predefined to be called for the processing and analyzing the molecular diagnostic amplification data among the sub-modules processes and analyzes the molecular diagnostic amplification data for the plurality of target nucleic acid molecules by referencing the parameter value in response to a request of the target presence/absence determining software.

In accordance with another embodiment of a method for distrubutinging an assay component performed by a device for generating an assay component used in target presence/absence determining software that displays molecular diagnostic amplification data, the method comprising: acquiring molecular diagnostic amplification data generated using a molecular diagnostic assay for a plurality of target nucleic acid molecules; generating the assay component based at least partially on the molecular diagnostic amplification data; distributing the assay component to a target presence/absence determining terminal on which the target presence/absence determining software is stored, wherein the assay component includes: a processing/analysis module including sub-modules for processing and analyzing the molecular diagnostic amplification data for the plurality of target nucleic acid molecules in the target presence/absence determining software, a parameter value referenced by the sub-modules, and wherein a sub-module predefined to be called for the processing and analyzing the molecular diagnostic amplification data among the sub-modules processes and analyzes the molecular diagnostic amplification data for the plurality of target nucleic acid molecules by referencing the parameter value in response to a request of the target presence/absence determining software.

In accordance with another embodiment of a method performed by a target presence/absence-determining terminal for determining target presence/absence using an assay component used in used in target presence/absence-determining software displaying molecular diagnostic amplification data, the method comprising: obtaining an assay component generated at least partially based on molecular diagnostic amplification data generated using a molecular diagnostic assay for a plurality of target nucleic acid molecules; and processing/analysis the molecular diagnostic amplification data for the plurality of target nucleic acid molecules using the assay component, wherein the assay component comprises: a processing/analysis module including submodules for processing and analyzing the molecular diagnostic amplification data for the plurality of target nucleic acid molecules in the target presence/absence-determining software; and a parameter value referenced by the sub-modules, and wherein one or more of the submodules, which are predefined to be referenced during processing and analysis of the molecular diagnostic amplification data, process and analyze the data for the plurality of target nucleic acid molecules by referencing the parameter value in response to a request from the target presence/absence-determing software.

In accordance with another embodiment of a a device for generating an assay component used in target presence/absence-determining software displaying molecular diagnostic amplification data, the device comprising: a processor; a memory; and a computer program loaded in the memory and executed by the processor, wherein the computer program comprises: instructions for obtaining molecular diagnostic amplification data generated using a molecular diagnostic assay for a plurality of target nucleic acid molecules; and instructions for generating the assay component at least partially based on the molecular diagnostic amplification data, instructions for distributing the assay component to a terminal on which the target presence/absence-determining software is stored, wherein the assay component comprises: a processing/analysis module including sub-modules for processing and analyzing the molecular diagnostic amplification data for the plurality of target nucleic acid molecules in the target presence/absence-determining software; and a parameter value referenced by the sub-modules, and wherein one or more of the submodules, which are predefined to be referenced during processing and analysis of the molecular diagnostic amplification data, process and analyze the data for the plurality of target nucleic acid molecules by referencing the parameter value in response to a request from the target presence/absence-determing software.

In accordance with another embodiment of a device for generating an assay component used in target presence/absence-determining software displaying molecular diagnostic amplification data, the device comprising: a processor; a memory; and a computer program loaded in the memory and executed by the processor, wherein the computer program comprises: instructions for obtaining molecular diagnostic amplification data generated using a molecular diagnostic assay for a plurality of target nucleic acid molecules; and instructions for generating the assay component at least partially based on the molecular diagnostic amplification data, instructions for distributing the assay component to a terminal on which the target presence/absence-determining software is stored, wherein the assay component comprises:
a processing/analysis module including sub-modules for processing and analyzing the molecular diagnostic amplification data for the plurality of target nucleic acid molecules in the target presence/absence-determining software; and a parameter value referenced by the sub-modules, and wherein one or more of the submodules, which are predefined to be referenced during processing and analysis of the molecular diagnostic amplification data, process and analyze the data for the plurality of target nucleic acid molecules by referencing the parameter value in response to a request from the target presence/absence-determing software.

### [Advantageous Effect]

According to an embodiment, an assay component including a processing/analysis module and a parameter value referenced by the processing/analysis module may be generated and distributed for each molecular diagnostic assay.

According to an embodiment, it is possible to efficiently deploy and manage a deployed processing/analysis module and a parameter value by an assay component separated from target presence/absence determining software.

### [Description of Drawings]

FIG. 1 is a flowchart of a method for generating an assay component according to an embodiment.
FIG. 2 conceptually illustrates a nucleic acid detection device and an assay component optimization device that are connected to a device for generating an assay component according to an embodiment.
FIG. 3 is a block diagram of the device for generating an assay component according to an embodiment.
FIG. 4 is a diagram for describing the assay component generated for each molecular diagnostic assay according to an embodiment.
FIG. 5 is a diagram illustrating an example of the processing/analysis module, the parameter, and the assay information that are included in the assay component according to an embodiment.
FIG. 6 is a diagram illustrating an example of target-specific processing/analysis modules, parameters, and assay information included in the assay component according to an embodiment.
FIG. 7 is a flowchart of a method for generating an assay component for an assay according to another embodiment.
FIG. 8 is a diagram schematically illustrating a network relationship between a device for generating an component and the target presence/absence determining terminal according to an embodiment.

### [Mode for Disclosure]

The advantages and features of the embodiments and the methods of accomplishing the embodiments will be clearly understood from the following description taken in conjunction with the accompanying drawings. However, embodiments are not limited to those embodiments described, as embodiments may be implemented in various forms. It should be noted that the present embodiments are provided to make a full disclosure and also to allow those skilled in the art to know the full range of the embodiments. Therefore, the embodiments are to be defined only by the scope of the appended claims.

In describing embodiments of the present invention, if it is considered that a detailed description of a known function or configuration may unnecessarily obscure the gist of the present invention, the detailed description will be omitted. In addition, the terms described below are terms defined in consideration of functions in the embodiments of the present invention, the terms may vary according to the intention or precedent of a technician working in the field, the emergence of new technologies, and the like. Therefore, the terms used in the present disclosure should be defined based on the meaning of the terms and the overall contents of the present disclosure, not just the name of the terms.

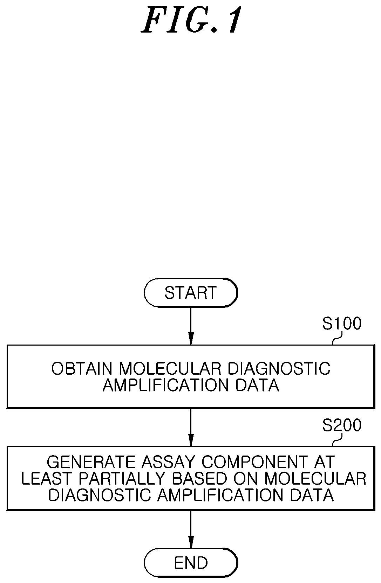

Before explaining Fig. 1, the terms used herein will be described.

The term "sample" includes biological samples (e.g., cells, tissues, and body fluids) and non-biological samples (e.g., food, water, and soil). Among the samples, the biological samples may include at least one of, for example, viruses, bacteria, tissues, cells, blood (including whole blood, plasma, and serum), lymph, bone marrow fluid, saliva, sputum, swab, aspiration, milk, urine, stool, eye fluid, semen, brain extracts, spinal fluid, joint fluid, thymic fluid, bronchoalveolar lavage fluid, ascites, or amniotic fluid. These samples may or may not include the above-described target nucleic acid molecules.

Meanwhile, when the target nucleic acid molecules described above are nucleic acid molecules or include the nucleic acid molecules, a nucleic acid extraction process known in the art may be performed on a sample estimated as including the target nucleic acid molecules (reference: Sambrook, J. et al., Molecular Cloning. A Laboratory Manual, 3rd ed. Cold Spring Harbor Press (1101)). The nucleic acid extraction process may vary depending on a type of samples. In addition, when the extracted nucleic acid is RNA, the extracted nucleic acid may additionally undergo a reverse transcription process to synthesize cDNA (reference: Sambrook, J. et al., Molecular Cloning. A Laboratory Manual, 3rd ed. Cold Spring Harbor Press (1101)).

The term "target" includes various substances (e.g., biological substances and non-biological substances), which may refer to the same subject as the term "target nucleic acid molecules" or "target analyte".

The target may specifically include the biological substances, and more include specifically at least one of nucleic acid molecules (e.g., DNA and RNA), proteins, peptides, carbohydrates, lipids, amino acids, biological compounds, hormones, antibodies, antigens, metabolites, or cells. The target may include a target nucleic acid molecule.

The term "target of interest" may refer to a subject to be read in a presence/absence determining process among "targets."

The term "cycle" refers to a unit of change in conditions in performing multiple measurements accompanied by a change in certain conditions. The change in certain conditions means an increase or decrease in, for example, temperature, reaction time, number of reactions, concentration, pH, or number of times of replications of a measurement subject (e.g., nucleic acid). Therefore, the cycle may be a time or a process cycle, a unit operation cycle, and a reproductive cycle.

More specifically, the term "cycle" means one unit of repetition when a reaction of a certain process is repeated or is repeated based on a certain time interval.

Alternatively, the term "cycle" may mean one unit of repetition when a certain action is repeated according to the progress of the reaction.

For example, when a nucleic acid amplification reaction is performed, an action of detecting a signal generated at a certain time interval may be repeated, and may mean one unit of repetition. In this case, the cycle may have a unit of time.

For example, in the case of the nucleic acid amplification reaction, one cycle means a reaction that includes a denaturation step of a nucleic acid, an annealing step of a primer, and an extension step of a primer. In this case, the change in certain conditions means the increase in the number of times of repetitions of the reaction and the repeating unit of a reaction involving a series of steps is designated as one cycle. The cycle number may include the number of times of reactions or the reaction time.

Meanwhile, an amplification reaction for amplifying a signal representing the presence of a target (target nucleic acid molecules or target of interest) may be performed in a method (e.g., real-time PCR method) in which the signal is also amplified while the target is amplified. Alternatively, according to an embodiment, the amplification reaction may be performed in a method in which only the signal representing the presence of the target is amplified without the target being amplified (e.g., CPT method (Duck P, et al., Biotechniques, 9:142-148 (1990)), Invader assay (US Pat. Nos. 6,358,691 and 6,194,149)).

Meanwhile, the above-described target or target analyte, particularly the target nucleic acid molecules, may be amplified by various methods: a polymerase chain-reaction (PCR), a ligase chain reaction (LCR) (U.S. Pat. Nos. 4,683,195 and 4,683,202; PCR Protocols: A Guide to Methods and Applications (Innis et al., eds., 1990)), strand displacement amplification (SDA) (Walker, et al. Nucleic Acids Res. 20(7):1691-6 (1992); Walker PCR Methods Appl 3(1):1-6 (1993)), transcription-mediated amplification (Phyffer, et al., J. Clin. Microbiol. 34:834-841 (1996); Vuorinen, et al., J. Clin. Microbiol. 33:1856-1859 (1995)), nucleic acid sequence-based amplification (NASBA) (Compton, Nature 350(6313):91-2 (1991)), rolling circle amplification (RCA) (Lisby, Mol. Biotechnol. 12(1):75-99 (1999); Hatchet et al., Genet. Anal. 15(2):35-40 (1999)) and Q-beta replicase (Lizardi et al., BiolTechnology 6:1197(1988)), loop-mediated isothermal amplification (LAMP, Y. Mori, H. Kanda and T. Notomi, J. Infect. Chemother., 2013, 19, 404-411), recombinase polymerase amplification (RPA, J. Li, J. Macdonald and F. von Stetten, Analyst, 2018, 144, 31-67), etc.

Meanwhile, the amplification reaction amplifies the signal while the amplification of the target (specifically, the target nucleic acid molecules) is accompanied. For example, the amplification reaction is performed using PCR, specifically real-time PCR, or an isothermal amplification reaction (e.g., LAMP or RPA).

Here, the term "signal value" means a value that is quantified according to a certain scale of a level of a signal (e.g., signal intensity) actually measured in a cycle of a signal generation reaction, particularly an amplification reaction, or modified values thereof. The modified values may include a mathematically processed signal value of an actually measured signal value. Examples of the mathematically processed signal values of the actually measured signal value (i.e., a signal value of a raw data set) may include a logarithm or derivatives.

The term " target determining presence/absence determining process" means an operation of determining presence/absence of a target in a sample using a signal value, and further includes operations of analyzing data used to determine the presence/absence of the target.

Hereinafter, various implementation examples of the present disclosure will be described with reference to the drawings.

FIG. 1 is a flowchart of a method for generating an assay component according to an embodiment.

Referring to FIG. 1, in step S100, molecular diagnostic amplification data generated using a molecular diagnostic assay of a plurality of target nucleic acid molecules may be acquired.

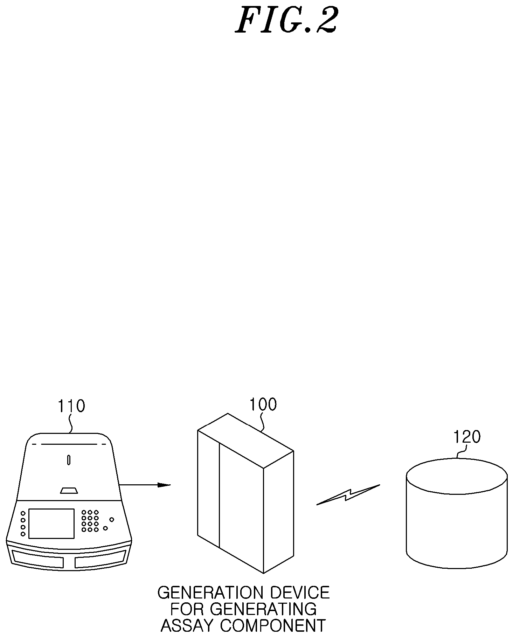

In an embodiment, the molecular diagnostic assay may refer to a detection reagent used for molecular diagnosis. The molecular diagnostic amplification data generated using the molecular diagnostic assay will be described in detail with reference to FIG. 2.

The method for generating an assay component according to an embodiment may be performed by a device 100 for generating an assay component used in target presence/absence determining software that displays molecular diagnostic amplification data. The device 100 for generating an assay component is a device that acquires molecular diagnostic amplification data and generates an assay component using the molecular diagnostic amplification data.

In step S200, the assay component may be generated based at least partially on the molecular diagnostic amplification data.

The assay component is a configuration used when performing a target presence/absence determining operation in the target presence/absence determining software, and may include at least one of a processing/analysis module, parameter values, or assay information.

Here, the processing/analysis module is a module for processing and analyzing the molecular diagnostic amplification data for the plurality of target nucleic acid molecules in the target presence/absence determining software.

The target presence/absence determining software is software that may apply the acquired molecular diagnostic amplification data to one or more processing/analysis modules for determining in a pre-designated order to determine the presence/absence of the target nucleic acid molecules. In an embodiment, the target presence/absence determining software may be a program installed in a target presence/absence determining terminal.

The target presence/absence determining software may include a function for displaying an operation result of the assat component. For example, the target presence/absence determining software may be a program that may display the results of processing and analysis performed in the component for an assay as at least one of a number, a chart, or a graph.

The processing/analysis module includes sub-modules. In an embodiment, the sub-modules may be algorithms or software. Among these sub-modules, a sub-module called when processing and analyzing the molecular diagnostic amplification data may be predefined. For example, when processing and analyzing molecular diagnostic data is performed, all the sub-modules may be called, some necessary sub-modules may be called, or only one sub-module may be called.

The pre-defined sub-modules may process and analyze the molecular diagnostic amplification data for the plurality of target nucleic acid molecules by referring to parameter values. In this case, the pre-defined sub-modules may process and analyze the molecular diagnostic amplification data on a presence/absence determining terminal according to a request of the target presence/absence determining software.

The parameter value may be referenced by the sub-modules. The definition of the parameter value will be described below.

In the presence/absence determining terminal, the molecular diagnostic amplification data for the plurality of target nucleic acid molecules may be processed and analyzed by referring to the parameter value. In the presence/absence determining terminal, the operation of determining the presence/absence of the target nucleic acid molecules may be performed by using the processing/analysis module, the parameter values, and the assay information that are included in the assay component.

FIG. 2 conceptually illustrates a nucleic acid detection device and an assay component optimization device that are connected to a device for generating an assay component according to an embodiment.

FIG. 2 illustrates the device 100 for generating an assay component that generate an assay component, a nucleic acid detection device 110, and a component optimization device 120 according to an embodiment. These devices 100, 110, and 120 may be connected to each other by wired or wireless communication. However, FIG. 2 is merely exemplary, and the idea of the present disclosure is not limited to what is illustrated in FIG. 2.

Meanwhile, unlike what was described above, the assay component optimization device 120 and the target presence/absence determining software may be implemented by being included in the device 100 for generating an assay component. However, the following description will be made on the premise that the above-described devices 100, 110, and 120 are implemented or connected as illustrated in FIG. 1. Hereinafter, each assay component will be described in detail.

The nucleic acid detection device 110 is implemented to perform a nucleic acid amplification reaction and a nucleic acid detection operation on a sample. According to the embodiment, the nucleic acid detection device 110 may be implemented to perform the nucleic acid detection operation without performing the nucleic acid amplification reaction. However, the following description will be made on the premise that the nucleic acid detection device 110 is implemented to perform the nucleic acid amplification reaction as well.

Meanwhile, as the above-described nucleic acid amplification reaction is performed, when a sample in a reaction vessel includes target nucleic acid molecules, not only is the amount of the target nucleic acid molecules amplified, but a magnitude of a signal generated by the above-described signal generation means may also be amplified as described above. Since the magnitude of the signal generated by the signal generation means may also be amplified when the nucleic acid amplification reaction is performed, the nucleic acid amplification reaction may cause a signal generation reaction as described above.

Accordingly, the nucleic acid detection device 110 is implemented to detect the magnitude of the signal. Specifically, the nucleic acid detection device 110 may monitor in real time the magnitude of the signal that changes according to the progress of the nucleic acid amplification reaction. The monitored result may be output from the nucleic acid detection device 110 in the form of the data set as mentioned in the definition of the term, for example, as the intensity of the signal per cycle. Here, the intensity of the signal per cycle is information that serves as a basis for determining the presence/absence of the target nucleic acid molecules in the corresponding sample. The nucleic acid detection device 110 may generate the molecular diagnostic amplification data using a molecular diagnostic assay of a plurality of target nucleic acid molecules.

The molecular diagnostic amplification data may be acquired from a result of a nucleic acid polymerase chain-reaction (PCR) targeting the plurality of target nucleic acid molecules.

The device 100 for generating an assay component may acquire the molecular diagnostic amplification data generated using the molecular diagnostic assay of the plurality of target nucleic acid molecules from the nucleic acid detection device 110.

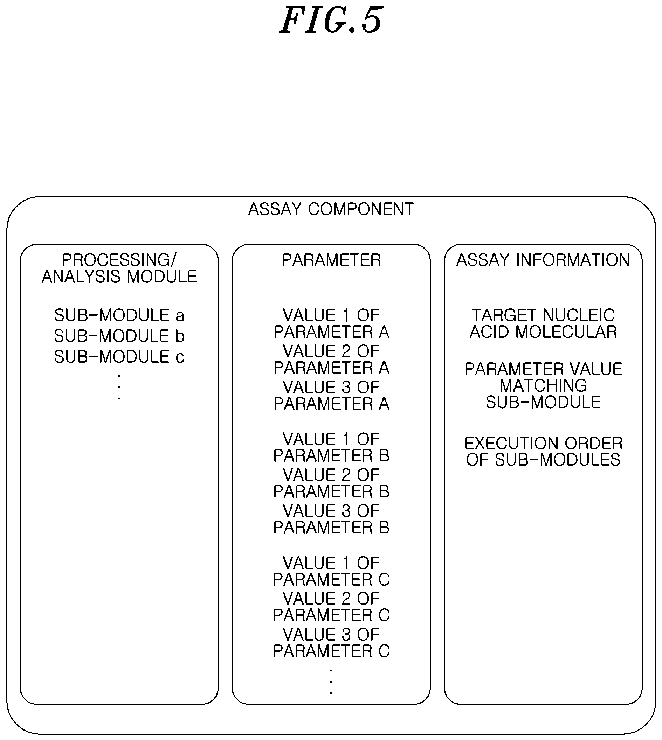

The component optimization device 120 may be implemented by a PC, a server, etc. The component optimization device 120 may optimize configurations included in the assay component. The component optimization device 120 may optimize the processing/analysis module used for processing or analyzing the molecular diagnostic amplification data, parameter values, or an execution order of the processing/analysis module. Here, a specific example of the processing/analysis module, the parameter values, or the execution order of the processing/analysis module are described in detail with reference to FIG. 5.

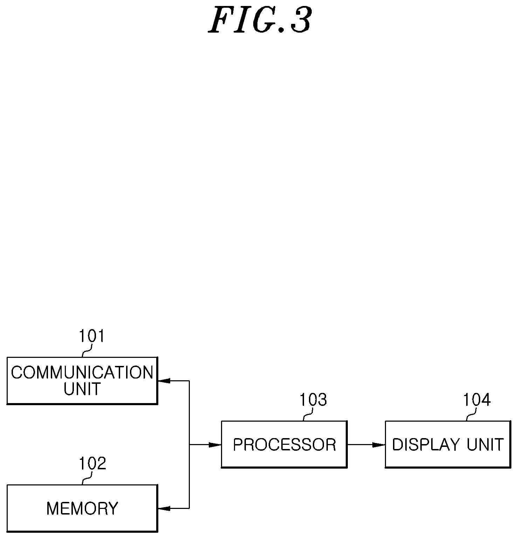

Hereinafter, the device 100 for generating an assay component illustrated in FIG. 2 will be described in more detail with reference to FIG. 3.

FIG. 3 is a block diagram of the device for generating an assay component according to an embodiment. Referring to FIG. 3, the device 100 for generating an assay component includes, but is not limited thereto, a communication unit 101, a memory 102, a processor 103, and a display unit 104.

First, the communication unit 101 is implemented as a wired or wireless communication module. The device 100 for generating an assay component may perform communication with the outside through the communication unit 101. For example, the device 100 for generating an assay component may receive the molecular diagnostic amplification data regarding the amplification result from the nucleic acid detection device 110 through the communication unit 101. In addition, the device 100 for generating an assay component may receive information required for generating the assay component from the outside, for example, the assay component optimization device 120 of FIG. 2.

Various types of data or information including at least one command are stored in the memory 102. The data to be stored may include data received from the assay component optimization device 120 through the communication unit 101 or data processed by the processor 103, and these data or information may include various types of information required for generating the assay component.

Meanwhile, in FIG. 3, the memory 102 is illustrated as a separate configuration from the processor 103, but the memory 102 and the processor 103 may be integrated into a single device. For example, the memory 102 may be a storage such as a cache included inside the processor 103.

The display unit 104 may be driven by the processor 103 as follows.

First, information may be displayed on the display unit 104. In addition, a user may input predetermined information through the display unit 104. For such display or input, the display unit 104 may be implemented as a touch screen or a touch pad, and in contrast, may be implemented through a combination of an LCD monitor and a keyboard, etc. Here, the information displayed on the display unit 104 may be transmitted from the outside to the device 100 for generating a component for an assay through the communication unit 101 or loaded from the memory 102, but is not limited thereto.

According to an embodiment, an input screen for generating the component for an assay is displayed on the display unit 104. Specifically, an input screen for receiving a type or order of modules, parameter values based on the modules, etc., is displayed. The type or order of modules and parameter values included in this screen may be transmitted from the outside, such as the component optimization device 120, to the device 100 for generating a component for an assay through the communication unit 101, or loaded from the memory 102, but is not limited thereto.

Next, the processor 103 may be implemented by a central processing unit (CPU), a graphics processing unit (GPU), a micro controller unit (MCU), or a dedicated processor in which the methods according to the embodiments are performed. Hereinafter, the processor 103 may collectively refer to a single processor or multiple processors, for example, a multi-core processor.

The processor 103 may write data to the memory 102. In addition, the processor 103 may execute the command stored in the memory 102. For example, the processor 103 executes the command stored in the memory 102, thereby performing the following operations by the device 100 for generating a component for an assay.

First, the molecular diagnostic amplification data is acquired from the nucleic acid detection device 110.

Second, a component for an assay for molecular diagnostic is generated based at least partially on the acquired molecular diagnostic amplification data.

The molecular diagnostic amplification data may be acquired from a result of a nucleic acid polymerase chain-reaction (PCR) targeting the plurality of target nucleic acid molecules.

In an embodiment, the molecular diagnostic amplification data may be data acquired through real-time amplification.

The molecular diagnostic amplification data should be generated according to a molecular diagnostic development protocol during the development process of the detection reagent. According to an embodiment, the device 100 for generating a component for an assay may receive experimental information on an experiment that is the basis of the molecular diagnostic amplification data through the input screen.

For example, in the input screen, the name of the target nucleic acid molecules for which the component for an assay is to be generated, an experimenter, a configuration of a panel in which each of the plurality of candidate oligonucleotides for each well is received and the amplification reaction is performed in the nucleic acid detection device 110, and experimental information on PCR components, PCR protocols, PCR equipment, and PCR consumables may be input.

Specifically, experimental information on at least one experimental item group selected from among experimental item groups including the type of performance verification experiment to be performed on candidate oligonucleotides for detection of target nucleic acid molecules, materials used in the experiment, experimental procedures in the experiment, experimental equipment, and a combination thereof is input through the input screen.

In this case, the candidate oligonucleotide is used to detect the target nucleic acid in the target nucleic acid molecules by the nucleic acid polymerase chain-reaction (PCR), and means a subject of a test for whether the detection performance of the target nucleic acid molecules satisfies a predetermined level or for optimizing the detection performance.

By providing the input screen for inputting such experimental information, the device 100 for generating a component for an assay according to an embodiment may receive the experimental information required according to the molecular diagnostic development protocol during the detection reagent development process.

Next, in the device 100 for generating a component for an assay according to an embodiment, the data set is uploaded from the nucleic acid detection device 110 that performs an amplification reaction on a prepared reaction vessel based on the configuration information for the panel input through the input screen. That is, the device 100 for generating a component for an assay acquires the molecular diagnostic amplification data generated using the detection reagent for the target nucleic acid molecules.

The device 100 for generating a component for an assay generates the component for an assay for molecular diagnostics based at least partially on the acquired molecular diagnostic amplification data.

The component for an assay for molecular diagnostics is used for processing and analyzing the molecular diagnostic amplification data.

The display unit of the target presence/absence determining software may display data sets, noise-removed data, amplification curves, etc., for the molecular diagnostic amplification data.

In addition, the display unit of the target presence/absence determining software may display whether the target nucleic acid molecules are present or absent for each sample, and may display detection results such as a Ct value, a signal value (e.g., an RFU value) for each sample.

In addition, the display unit of the target presence/absence determining software may display a list of detection reagents according to target nucleic acid molecules of interest and their compositions. In this case, each detection reagent in the list of detection reagents may be assigned the generated component for an assay. Alternatively, the list of detection reagents for which the component for an assay is to be generated may be displayed in a waiting state.

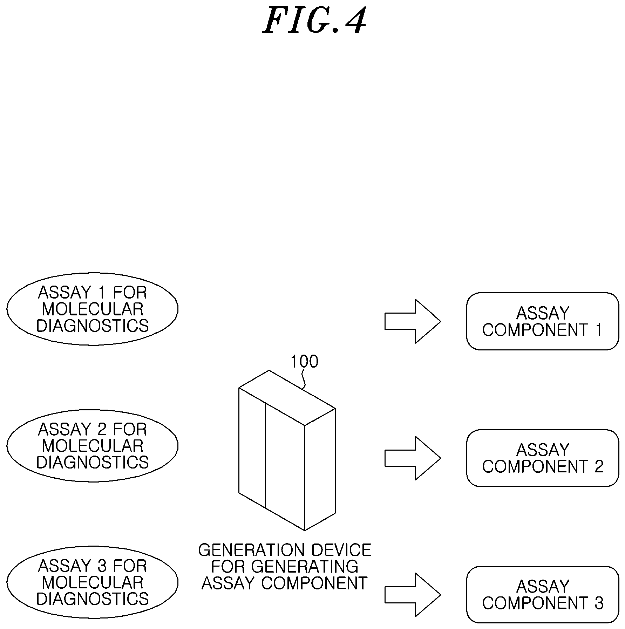

FIG. 4 is a diagram for describing the component for an assay generated for each molecular diagnostic assay according to an embodiment.

As illustrated in FIG. 4, the device for generating a component for an assay may generate a component for an assay differently for each molecular diagnostic assay.

For example, in order to generate the component for an assay for molecular diagnostic assays 1 to 3, the device for generating a component for an assay may individually generate component for assay 1 for molecular diagnostic assay 1, component for assay 2 for molecular diagnostic assay 2, and component for assay 3 for molecular diagnostic assay 3.

The processing/analysis module of the component for an assay for each molecular diagnostic assay is based on characteristics of a target drug, and therefore, is different for each molecular diagnostic assay. This is because the order of the sub-modules included in the processing/analysis module, and some sub-modules are added or excluded depending on the characteristics of the detection reagent. Even when the processing/analysis modules of some molecular diagnostic assays are the same, the parameter values of the processing/analysis modules may be different.

FIG. 5 is a diagram illustrating an example of the processing/analysis module, the parameter, and the assay information that are included in the component for an assay according to an embodiment.

As illustrated in FIG. 5, the component for an assay may include the processing/analysis module, the parameter values, and the assay information. The component for an assay exists as a single assembly in which the processing/analysis module and the parameter values for each molecular diagnostic assay are packed.

The processing/analysis module may include at least one sub-module. As illustrated in FIG. 5, the processing/analysis module may include sub-module a, sub-module b, and sub-module c.

In an embodiment, the processing/analysis module may be a common module for at least two target nucleic acid molecules among the plurality of target nucleic acid molecules.

The common module may include common sub-modules that are commonly used for the at least two target nucleic acid molecules among the plurality of target nucleic acid molecules. That is, the component for an assay may include sub-modules that are commonly used by each of the plurality of target nucleic acid molecules.

The sub-module may be various modules for processing and analyzing. In an embodiment, the sub-modules may include an algorithm that analyzes or mathematically processes a data set acquired during an amplification of the plurality of target nucleic acid molecules.

For example, the sub-modules may include an algorithm that performs an operation of outputting a cycle number when a data set acquired during an amplification of the plurality of target nucleic acid molecules reaches a threshold value and an operation of determining presence/absence of the target nucleic acid molecules based at least partially on the cycle number. Here, the cycle number may be defined as the number of times of reactions or time.

Also, the sub-modules may include an algorithm that performs one of (i) analyzing signals detected at a relatively high detection temperature and a relatively low detection temperature during an amplification of the plurality of target nucleic acid molecules, (ii) normalizing a plurality of data set acquired during the amplification of the plurality of target nucleic acid molecules, (iii) determining presence/absence of the target nucleic acid molecules using a parameter representing a maximum derivative of a sigmoid fitting curve for the data set acquired during the amplification of the plurality of target nucleic acid molecules, (iv) evaluating a shape of the sigmoid fitting curve using a value representing a profile of the data set acquired during the amplification of the plurality of target nucleic acid molecules, (v) performing regression analysis on the data set acquired during the amplification of the plurality of target nucleic acid molecules, and (vi) performing regression model analysis on a portion of the data set acquired through a signal generation reaction for the target nucleic acid molecules.

The sub-module may be a sub-module that subtracts a baseline signal included in the molecular diagnostic amplification data, a sub-module that acquires a predetermined curve by fitting the molecular diagnostic amplification data to a non-linear function, and a module that reads the presence/absence of the target nucleic acid molecules from the acquired curve.

Specifically, the sub-module that subtracts the baseline signal included in the molecular diagnostic amplification data includes a module that acquires corrected data by applying a normalization coefficient disclosed in WO 2017/086762 of the present applicant to signal values.

The normalization coefficient is provided using a reference value, a reference cycle, and a data set, the reference cycle is selected from the cycles of the data set, the reference value is an arbitrarily determined value, the normalization coefficient is provided by determining the relationship between the signal value of the cycle corresponding to the reference cycle in the data set and the reference value, and the normalization coefficient is applied to the signal values of the raw data set to acquire corrected signal values and provide the corrected signal values to the raw data set. For example, the reference value is a value used to provide the normalization coefficient. In this specification, the reference value means an arbitrary value applied to the reference cycle to correct the signal values of the data set. The same reference value is applied to data sets to be normalized by the same reference. When the data sets to be corrected are a plurality of data sets, the plurality of data sets may be corrected by the same reference value. The reference value may be an arbitrarily determined value. Preferably, the reference value is an arbitrarily determined value among non-zero real numbers. The reference value may be arbitrarily selected by the experimenter as long as the presence of the target nucleic acid molecules in the sample may be determined by the correction data set using the reference value. Therefore, the reference value to be used for correction of the data set may be determined within a range of signal values that may be acquired in the reference cycle by the same kind of signal generation reaction as the signal generation reaction from which the data set was acquired. The reference value may be acquired separately from the data set to be corrected. Specifically, the reference value may be determined from the data set acquired from the signal generation reaction for the same kind of target nucleic acid molecules as the target nucleic acid molecules to be analyzed. Alternatively, the reference value may be acquired from a group of data sets including the data set to be corrected. Preferably, the reference value may be a value of the same kind as the signal value of the data set to be corrected, and may be in the same unit or dimension as the data set to be corrected. However, even if the reference value and the signal value of the data set have different units or dimensions, or even if the reference value does not have units or dimensions, appropriate normalization coefficients for each reaction are provided from the reference value and the signal value of the data set to be corrected, and the correction data set may be acquired using the normalization coefficient for each reaction.

Next, the sub-module that acquires a predetermined curve by fitting the molecular diagnostic amplification data to the non-linear function may perform the analysis for determining the presence/absence of the target nucleic acid molecules using DSP (WO2019/066572) technology.

The parameters that are set using the DSP technology include parameters used in the performance data set processed by mathematically processing the raw data set. Here, the processed performance data set includes not only the data set acquired by mathematically processing the raw data set, but also the data acquired by mathematically processing the data acquired in this way again. For example, the processed performance data set is data including a first derivative of the raw data set and second and third derivatives, etc., acquired from the derivative.

The parameters of the DSP may be applied to a function for setting the Ct parameter value described above. For example, the DSP uses the fitting accuracy of the non-linear function for the data set as a direct indicator for analyzing the target analyte, acquires the data set for the target nucleic acid molecules from a signal-generating reaction using a signal-generating means to analyze the target nucleic acid molecules without false positive and false negative results, especially false positive results, corrects the acquired data set including a plurality of data points including cycle numbers and signal values, generates the non-linear function for the corrected data set to determine the fitting accuracy of the non-linear function for the corrected data set, and determines the presence/absence of the target analyte in the sample using the fitting accuracy. This analysis method requires various parameters and may be optimized according to the values set for each parameter. Using DSP (WO2019/066572) technology, the parameters used to read the presence/absence of the target nucleic acid molecules are assigned, and the analysis is performed by setting the DSP parameter values.

Next, the sub-module for determining the presence/absence of the target nucleic acid molecules from the curve acquired by fitting the molecular diagnostic amplification data to the non-linear function may include a module for extracting only data for each target nucleic acid from data acquired for the plurality of target nucleic acids at multiple different temperatures. For example, the modules disclosed in WO2015-147370, WO2015/147412, WO2015/147382 and WO2016/093619 of the present applicant that use multiple target detection temperatures may be used to assign parameters used in the process of determining the presence/absence of the target nucleic acid molecules, and the assigned parameter values may be set to analyze the data of the raw data set.

Even in the case of performing the analysis by setting values for parameters of a technology that uses the DSP and the multiple target detection temperatures, the parameter values may be adjusted and set through comparative analysis based on ground truth data labeled as positive and negative, in the same way that the analysis is performed by setting the value for the Ct parameter. For example, the parameter values may be adjusted so that the samples labeled as positive and the samples labeled as negative in the ground truth data match the results of performing the analysis by setting the parameter values.

In an embodiment, the parameter value may be used for signal processing of the target presence/absence determining software or serves as a criterion for a determination made by the signal processing.

In an embodiment, the parameter value may be (i) a parameter value defining a criterion for determining whether a value of a data set acquired during an amplification of the plurality of target nucleic acid molecules exceeds a threshold value, or (ii) a parameter value defining a criterion for determining presence/absence of the target nucleic acid molecules using a cycle number at which the value of the data set acquired during the amplification of the plurality of target nucleic acid molecules reaches the threshold value. For example, the parameter may be used for the mathematical processing of the molecular diagnostic amplification data or as the criterion for determining the presence/absence of the target nucleic acid molecules in the sample. The criterion may include a threshold intensity value of a signal used to determine the threshold cycle (Ct) of the sample.

**In** an embodiment, the parameter value is any one of (i) a parameter value indicating a relationship between a magnitude of a signal detected at a relatively high detection temperature and a magnitude of a signal detected at a relatively low detection temperature in a data set acquired during an amplification of the plurality of target nucleic acid molecules, (ii) a parameter value defining a normalization coefficient for normalizing a plurality of data sets acquired during the amplification of the plurality of target nucleic acid molecules, (iii) a parameter value defining a criterion for determining absence of the target nucleic acid molecules using a maximum derivative value of a sigmoid fitting curve applied to the plurality of data sets acquired during the amplification of the plurality of target nucleic acid molecules, (iv) a parameter value defining a criterion for determining presence of the target nucleic acid molecules using a parameter representing the maximum derivative value of the sigmoid fitting curve applied to the plurality of data sets acquired during the amplification of the plurality of target nucleic acid molecules, (v) a parameter value defining a criterion for determining a shape of the sigmoid fitting curve applied to the plurality of data sets acquired during the amplification of the plurality of target nucleic acid molecules, (vi) a parameter value defining a start cycle required for performing regression analysis on the plurality of data sets acquired during the amplification of the plurality of target nucleic acid molecules, (vii) a parameter value representing a minimum cycle of a final cycle required for performing the regression analysis on the plurality of data sets acquired during the amplification of the plurality of target nucleic acid molecules, (viii) a parameter value defining a criterion for determining the absence of the target nucleic acid molecules using fitting accuracy acquired by applying a regression model applied to a portion of the plurality of data sets acquired during the amplification of the plurality of target nucleic acid molecules, (ix) a parameter value defining a criterion for determining the absence of the target nucleic acid molecules using fitting accuracy acquired by applying regression model analysis to all of the plurality of data sets acquired during the amplification of the plurality of target nucleic acid molecules, and (x) a parameter value defining a criterion for determining the absence of the target nucleic acid molecules using a mathematical processing of a maximum value and a minimum value or a mathematical processing of a value at a final cycle and a minimum value of the plurality of data sets acquired during the amplification of the plurality of target nucleic acid molecules.

These parameters may be referenced in the process of performing the processing and analyzing by the sub-module described above.

In an embodiment, the component for an assay may include the assay information. The assay information may include at least one of information describing the plurality of target nucleic acid molecules for the molecular diagnostic assay, information matching the sub-modules and the parameter values referenced by the sub-modules according to each target nucleic acid molecule, or information defining the execution order of the sub-modules for processing and analysis.

Information describing the plurality of target nucleic acid molecules for the molecular diagnostic assay may be information describing genes, analytes or the like of the plurality of target nucleic acid molecules. Such assay information may be used to display properties of the component for an assay in the target presence/absence determining software.

In addition, the information matching the sub-modules and the parameter values referenced by the sub-modules according to each target nucleic acid molecule may include information on which processing/analysis should be used and which parameter values should be referenced when performing the operation for each of the plurality of target nucleic acid molecules.

The information defining the execution order of sub-modules for processing and analysis may include information on the order in which the sub-modules should be executed for each target nucleic acid molecule. Here, the execution order may be predefined for each of the plurality of target nucleic acid molecules.

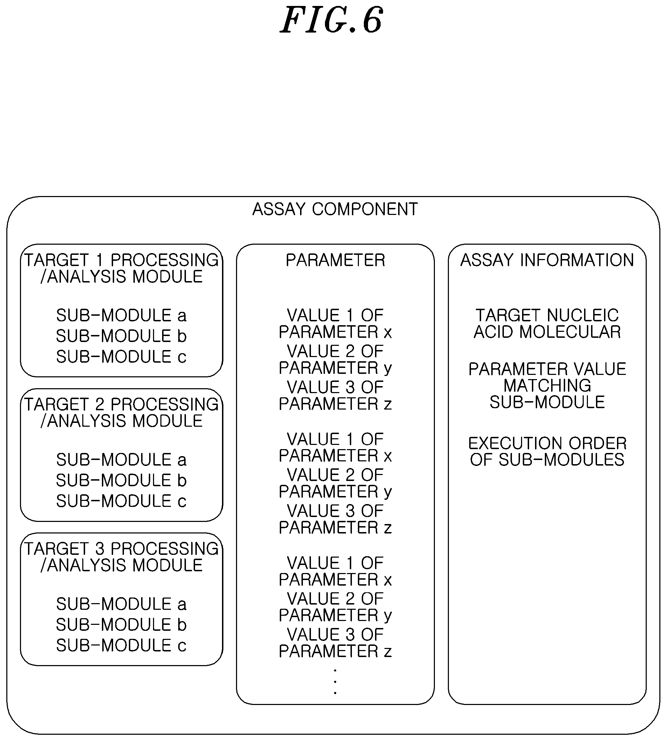

FIG. 6 is a diagram illustrating an example of target-specific processing/analysis modules, parameters, and assay information included in the component for an assay according to an embodiment.

As illustrated in FIG. 6, the processing/analysis module may be an individual module provided for each of the plurality of target nucleic acid molecules. That is, when the target nucleic acid molecules are target 1, target 2, and target 3, the processing/analysis module for the target 1, the processing/analysis module for the target 2, and the processing/analysis module for the target 3 may be different from each other. The individual module may include individual sub-modules that are individually used for each of the plurality of target nucleic acid molecules.

Also, when the component for the assay includes at least two processing/analysis modules, each of the at least two processing/analysis modules may include sub-modules that are at least partially identical to each other. Since some of the sub-modules may be implemented or fine-tuned differently for each target, some of the sub-modules may be the same or different for each target.

In an embodiment, the processing/analysis module may include a common module provided for some of the plurality of target nucleic acid molecules and an individual module provided for some of the plurality of target nucleic acid molecules.

Hereinafter, a method for optimizing a component for an assay will be described.

The component optimization device 120 described in FIG. 2 may perform 'optimization' on the processing/analysis module. The optimization may be designed in the process of optimizing the performance of the detection reagent.

Here, in the process of optimizing the performance of the detection reagent, a process of determining parameter values referenced in a pre-designated processing/analysis module is repeatedly performed until the performance of the detection reagent satisfies a predetermined criterion, and when the performance does not satisfy the criterion even after the repeated performance, a process of redesigning the pre-designated processing/analysis module to generate the processing/analysis module may be performed.

Here, the process of redesigning the pre-designated sub-modules may include at least one of modifying one or more sub-modules included in the pre-designated sub-modules, adding sub-modules not included in the pre-designated sub-modules to the component for the assay, and deleting at least one of the sub-modules or changing an execution order of the sub-modules when the pre-designated sub-modules include two or more sub-modules. For example, one or more sub-modules included in the pre-designated processing/analysis module may be modified when one or more sub-modules and/or parameter values need to be updated from an initial version, depending on the characteristics of the detection reagent.

For example, the first processing/analysis module needs to be updated to version 2 of a first processing/analysis module. A process of adding a sub-processing/analysis module that is not included in the pre-designated processing/analysis module to the component for an assay may be performed, for example, when a fourth processing/analysis module needs to be added to the component for an assay composed of the first processing/analysis module, the second processing/analysis module, and the third processing/analysis module. When the pre-designated processing/analysis module includes two or more sub-processing/analysis modules, a process of deleting at least one of the sub-processing/analysis modules may be performed, for example, when, for example, the second processing/analysis module is unnecessary in the component for an assay composed of the first processing/analysis module, the second processing/analysis module, or the third processing/analysis module. The process of changing the execution order of two or more sub-processing/analysis modules may be performed when the third processing/analysis module should be performed before the first processing/analysis module and the second processing/analysis module in the order of the first processing/analysis module, the second processing/analysis module, and the third processing/analysis module.

Meanwhile, the optimized processing/analysis module may be provided from the component optimization device 120 of FIG. 2 to the device 100 for generating a component for an assay. Here, the provision of the optimized processing/analysis module may be made in response to a request from the device 100 for generating a component for an assay.

Meanwhile, in the process of optimizing the processing/analysis module, the molecular diagnostic amplification data is required as described above. The molecular diagnostic amplification data may be acquired from the device 100 for generating a component for an assay, or may be acquired directly from the detection device 110.

In the device 100 for generating a component for an assay according to an embodiment, the molecular diagnostic amplification data generated using the detection reagent for the target nucleic acid molecules is acquired, and the component for an assay for molecular diagnosis is generated based at least partially on the molecular diagnostic amplification data acquired in this way.

Here, the component for an assay includes a processing/analysis module that is used by the target presence/absence determining software that displays the molecular diagnostic amplification data and/or the analysis results for the molecular diagnostic amplification data in the processing and analyzing of the molecular diagnostic amplification data, and the parameter values referenced by the processing/analysis module.

In this way, according to an embodiment, in the molecular diagnosis using the detection reagent, it may be easily managed through the components for an assay generated for each detection reagent which processing/analysis module should be applied to process or analyze the molecular diagnostic amplification data, and at this time, which parameters and their values should be referenced.

The device 100 for generating a component for an assay may generate the component for an assay for each molecular diagnostic assay.

The device 100 for generating a component for an assay performs experimental data analysis on a data set uploaded from the nucleic acid detection device 110 to generate the component for an assay for molecular diagnosis, and determines the processing/analysis module used for the presence/absence determining process of the corresponding target nucleic acid molecules.

The processing/analysis module may be a module that processes or analyzes the signal value acquired in the process of detecting the target nucleic acid molecules. Analyzing the signal value means, but is not limited to, determining the positive and negative for the presence/absence of the target nucleic acid molecules from the signal value, acquiring the Ct value as the signal value, or analyzing the end-relative fluorescence unit (end-RFU) as the signal value. Processing the signal value means, but is not limited to, mathematically changing or modifying the signal value.

Determining such a processing/analysis module requires considerable skill, and may cause considerable deviation and error, especially depending on the developer. In the present disclosure, the processing/analysis module and the parameter value most specialized for the corresponding detection reagent are designed by optimizing the processing/analysis module for each molecular diagnostic assay to generate the component for an assay.

To this end, the processing/analysis module that may optimize the performance of the detection reagent may be accurately acquired by converting the raw data set by the uploaded data set into the processed performance optimization data set.

The raw data set is an unprocessed data set initially acquired from the nucleic acid detection device 110, and various parameters may be used to convert the raw data set into the processed performance data set. Since the detection reagents have different parameters that may cause false positives or false negatives depending on their type, using the pre-designated parameter values may be inappropriate for some reactions, which may cause false positive or false negative results.

Therefore, it is necessary to perform accurate experimental data analysis on the uploaded data set to determine the processing/analysis module used in the process of determining the presence/absence of the corresponding target nucleic acid molecules. To this end, when analyzing the experimental data, parameters that cause the false positives or false negatives may be identified based on various factors such as the type and number of target nucleic acid molecules and the amplification reaction environment and conditions, and the processing/analysis module that finds the parameter values optimized for the detection reagent may be determined by setting the parameter values.

Here, the processing/analysis module includes, but is not limited to, processing/analysis software, a processing/analysis application, or a processing/analysis module, etc.

According to the present disclosure, such processing/analysis modules and parameter values are packed to exist for each molecular diagnostic assay, thereby generating the component for an assay for molecular diagnosis.

To this end, the device 100 for generating a component for an assay according to an embodiment may design the processing/analysis module and the parameter value most suitable for the detection reagent, and apply the an optimal processing/analysis modules and parameter values for each molecular diagnostic assay.

Each of the processing/analysis module and parameter value included in the component for an assay according to an embodiment is designed in the process of optimizing the performance of the detection reagent.

In order to optimize the performance of the detection reagent, the optimal processing/analysis module and parameter values are designed in this process. In the process of optimizing the performance of the detection reagent, the process of selecting the parameter value referenced in the pre-designated processing/analysis module is repeatedly performed until the performance of the detection reagent satisfies a predetermined criterion, and when the performance does not satisfy the criterion even after the repeated performance, the process of redesigning the pre-designated processing/analysis module to generate the processing/analysis module is performed.

In the device 100 for generating a component for an assay, the design of the processing/analysis module and parameter value to optimize the performance of the detection reagent depends on the results of the experimental data analysis, and the experimental data analysis may be performed as follows.

According to an embodiment, the experimental data analysis may be performed by comparing the prepared reaction vessel based on the configuration information for the panel on which the amplification reaction was performed with the ground truth data labeled as the positive and negative.

The ground truth data means the results actually identified by the experimenter who adjusted the concentration of the target nucleic acid molecules for each well of the reaction vessel, and the results of actual positive or negative determinations from actual clinical experiments (e.g., amplification reaction) may be used, but is not limited thereto.

For example, when analyzing the experimental data, the results of performing the experimental data analysis using the pre-designated processing/analysis module and the parameter values of the processing/analysis module are compared with the ground truth data, and whether the sample labeled as positive and the sample labeled as negative in the ground truth data match the results of the experimental data analysis may be compared and analyzed.

For the pre-designated processing/analysis module, the module and its parameter value may be predetermined. For example, a processing/analysis module and a parameter value for a respiratory detection reagent, a processing/analysis module and a parameter value for intestinal bacteria, or a processing/analysis module and a parameter value for sexually transmitted diseases may each be packaged, so a specific pre-designated processing/analysis module and parameter value may exist for each molecular diagnostic assay.

In the present disclosure, the molecular diagnostic amplification data acquired through the amplification reaction for the sample for which the ground truth data is provided may be applied to the pre-designated processing/analysis module and parameter value, and the results may be compared with the ground truth data to perform the experimental data analysis. Depending on the results of the experimental data analysis, it may be determined whether to generate a component for an assay using the pre-designated processing/analysis module and parameter value.

According to an embodiment, the device 100 for generating a component for an assay uses the pre-designated processing/analysis module and parameter value to generate the corresponding component for an assay for molecular diagnostic using the pre-designated processing/analysis module and parameter value when the result of the presence/absence determining process matches the ground truth data.

When the experimental data analysis result does not match the ground truth data through the pre-designated processing/analysis module and parameter value, or when a more efficient presence/absence determining is performed, or when an update to the pre-stored processing/analysis module and parameter value is required, the pre-designated processing/analysis module may be redesigned.

To this end, the device 100 for generating a component for an assay according to an embodiment may export experimental data (the molecular diagnostic amplification data) to the component optimization device 120 to request the processing/analysis module optimized for the detection reagent in order to optimize the processing/analysis module required for the presence/absence determining process of the corresponding detection reagent.

The component optimization device 120 stores the plurality of processing/analysis modules for analyzing the performance to set the optimal operating conditions in order to optimize the performance for the detection reagent.

The component optimization device 120 may analyze the experimental data (the molecular diagnostic amplification data) exported from the device 100 for generating a component for an assay, and determine the optimal processing/analysis module by identifying the processing/analysis module and/or parameter value that causes the false positive or the false negative under various conditions.

As a result of the experimental data analysis of the component optimization device 120, the device 100 for generating a component for an assay that receives the processing/analysis module optimized for the detection reagent generates the corresponding component for an assay for molecular diagnostic using the received processing/analysis module and parameter value.

In this way, the processing/analysis module and parameter value may be used in the process of performing the presence/absence determining, and the processing/analysis module and parameter value may differ depending on the type of target nucleic acid molecules or the type of compositions used for molecular diagnosis. The device 100 for generating a component for an assay according to the present disclosure may design the processing/analysis module and parameter value optimized for each molecular diagnostic assay and generate the designed processing/analysis module and parameter value as the component for an assay.

In this way, when using the target presence/absence determining software capable of identifying the presence/absence of the target nucleic acid molecules, it is not necessary to perform the overall validation of the target presence/absence determining software even if changes occur, such as the addition of the target nucleic acid molecules to be detected, or the addition/change of the type or order of new processing/analysis modules.

The conventional target presence/absence determining software is target presence/absence determining software that may identify the presence/absence of the target nucleic acid molecules for each molecular diagnostic assay in a list of multiple detection reagents. Since each detection reagent shares or links at least one presence/absence determining module required for determining, and performs the presence/absence determining through independent commands and branches for each molecular diagnostic assay, even when the processing/analysis module and/or parameter value is changed or added only for one of the multiple detection reagents, the verification should be performed again for multiple detection reagents to perform regulatory approval for the target presence/absence determining software that reflects the changed or added processing/analysis module and/or parameter value.

However, since the device 100 for generating a component for an assay according to an embodiment individually generates and distributes the components for an assay for each molecular diagnostic assay including the processing/analysis module and parameter value, when a change occurs to the target component for an assay or when the detection reagent is added due to the addition of the target nucleic acid molecules, the verification may be performed by separating each target component for an assay into individual units, so the overall verification of the target presence/absence determining software is not required.

According to an embodiment, the device 100 for generating a component for an assay generates the component for an assay by bundling the processing/analysis module and parameter value into a dedicated pack for each molecular diagnostic assay, so each molecular diagnostic assay may be accessed in individual units, enabling efficient updating of the update targets and distribution of update information.

The component for an assay may be generated for each detection reagent, generated in a version for parameter value setting, a version for performance verification of a prototype of detection reagents, a version for information identification for regulatory approval of detection reagents, and a version for result identification during a clinical stage of detection reagents, respectively, or generated for different target nucleic acid molecules.

For example, in the development process of detection reagents, the reagents development and selection of sophisticated oligonucleotides suitable for commercialization, along with various performance experiments, are essential. The version for parameter value setting is a component for an assay employed in the process of selecting the optimal oligonucleotide among from multiple candidate oligonucleotides during each performance experiment.

The version for performance verification of a prototype is a component for an assay used when reviewing the manufacturing quality for process errors during the manufacturing process of the target detection reagent.

The version for information identification for regulatory approval is a component for an assay for country-specific approval/certification for selling detection reagents as a medical device.

Since the version for result identification during a clinical stage may vary depending on various factors such as the type of template (artificial template or clinical specimen or strain supplied locally), the type of reaction vessel (plate, strip), and the concentration of the template, the version for result identification during a clinical stage is a component for an assay for the optimal processing/analysis module and parameter value of the parameter so that it may exhibit suitable performance even under these conditions and environmental changes.

Meanwhile, in the present disclosure, the target nucleic acid molecules include a plurality of different target nucleic acid molecules, and the processing/analysis module and parameter value may be provided for each different target nucleic acid molecule.

In this case, the molecular diagnostic amplification data acquired from the nucleic acid detection device 110 may exist for each detection channel. The detection channel selectively detects light in a specific wavelength range. As a result, only a specific optical label among the optical labels included in the sample generates an optical signal, and the optical signal is detected for each specific wavelength range. The optical label may be an optical label selected from the group consisting of, for example, FAM, HEX, CAL Fluor Red 610, Quasar 670, and Quasar 705.

In addition, when detecting the plurality of target nucleic acid molecules (low temperature/high temperature) with one detection channel and/or detecting the plurality of target nucleic acid molecules with multiple detection channels, the processing/analysis modules and parameter values may be provided for each of the plurality of target nucleic acid molecules.

According to an embodiment, the device 100 for generating a component for an assay generates a component for an assay for molecular diagnostic, and sets the processing/analysis modules and parameter values of the generated component for an assay so that the processing/analysis modules and parameter values may exhibit suitable performance even when applied to clinical data.

Since the results of the presence/absence detection may vary depending on various factors, such as the type of target nucleic acid molecules (artificial template, locally sourced clinical specimens, or standard strains), the type of reaction vessel (plate, strip), and the concentration of the target nucleic acid molecules, to ensure suitable performance under changing the conditions and environments, the analysis is performed on the results of the presence/absence detection with various factors applied, so the processing/analysis modules and parameter values of the generated component for an assay may be validated for various factors.

Even in this process, as described above, the most suitable processing/analysis module and parameter value are designed for each factor to apply the optimal processing/analysis module and parameter value. The process of selecting the parameter value referenced in the pre-designated processing/analysis module is repeatedly performed until the performance of the corresponding factor satisfies a predetermined criterion, and when the performance does not satisfy the criterion even after the repeated performance, the process of redesigning the pre-designated processing/analysis module to generate the processing/analysis module is performed.

The device 100 for generating a component for an assay may export experimental data (the molecular diagnostic amplification data) to the component optimization device 120 to request the processing/analysis module optimized for the detection reagent in order to optimize the processing/analysis module required for the presence/absence determining process of the corresponding factor.

As a result of the experimental data analysis of the component optimization device 120, the device 100 for generating a component for an assay, which receives the processing/analysis module optimized for the detection reagent, designs all the processing/analysis modules and parameter values applicable to various factors using the received processing/analysis module and parameter value, thereby generating the components for an assay, respectively.

The component for an assay for molecular diagnostic generated through this process is distributed to the target presence/absence determining terminal after performing the verification in the final target presence/absence determining software.

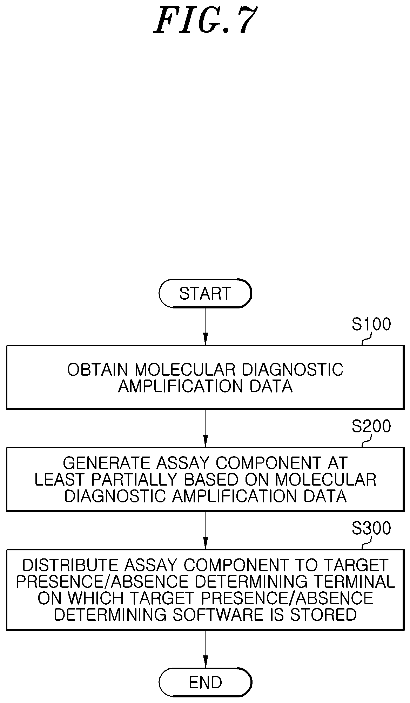

FIG. 7 is a flowchart of a method for generating a component for an assay according to another embodiment.

A method for generating a component for an assay according to another embodiment includes the same method according to the above-described embodiment, and different contents are described below.

Referring to FIG. 7, in step S100, molecular diagnostic amplification data generated using a molecular diagnostic assay of a plurality of target nucleic acid molecules may be acquired. In step S200, the component for an assay may be generated based at least partially on the molecular diagnostic amplification data. Thereafter, in step S300, the component for an assay may be distributed to the target presence/absence determining terminal on which the target presence/absence determining software is stored.

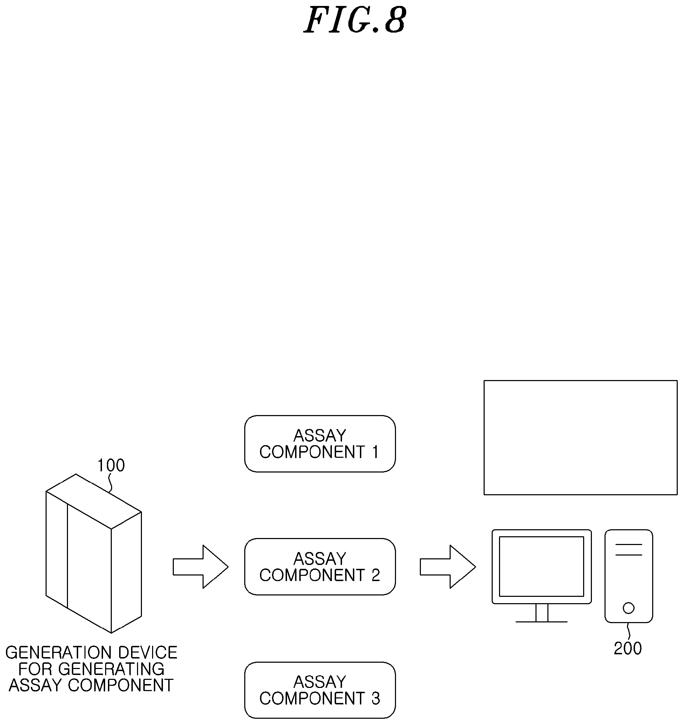

FIG. 8 is a diagram schematically illustrating a network relationship between a device for generating a component for an assay and the target presence/absence determining terminal according to an embodiment.

As illustrated in FIG. 8, the components for an assay generated for each molecular diagnostic assay may be deployed to the target presence/absence determining terminal 200. The processing/analysis module and processing and analysis results for the parameter values of each component for an assay for molecular diagnostic may be identified through the target presence/absence determining software of the target presence/absence determining terminal 200. In the display unit of the target presence/absence determining software of FIG. 8, the list of detection reagents to which each component for an assay is assigned is displayed, and the determining results of the detection reagents selected from the list are simultaneously displayed on the screen.

In this case, the component for an assay may be distributed for update. In an embodiment, the distribution for update may be performed to the terminal with the target presence/absence-determining software in order to address an issue of noise or abnormal signal removal in the molecular diagnostic amplification data that cannot be resolved by a pre-stored assay component existing in the target presence/absence determining terminal 200. For example, such distribution may be performed when the processing/analysis module or parameter value is a minor change such as noise removal or abnormal signal removal.

In an embodiment, the distribution for the update may overwrite the pre-stored component for an assay existing in the target presence/absence determining terminal 200, or the target presence/absence determining terminal 200 may be configured to include the component for an assay along with the pre-stored component for an assay existing in the target presence/absence determining terminal 200.

In an embodiment, the component for an assay distributed in this way may be installed in the target presence/absence-determining terminal 200 by a user authorized to install the component for an assay.

In an embodiment, the component for the assay may be deleted from the target presence/absence determining terminal 200 by a user who is authorized to delete the component for the assay.

Hereinafter, a method for determining target presence/absence using the component for an assay according to another embodiment will be described. The present embodiment may be performed by the target presence/absence determining terminal 200 that performs the target presence/absence determining.

First, the target presence/absence determining terminal 200 may acquire the component for an assay generated based at least partially on the molecular diagnostic amplification data generated using the molecular diagnostic assay of the plurality of target nucleic acid molecules.

The target presence/absence determining terminal 200 may process and analyze the molecular diagnostic amplification data for the plurality of target nucleic acid molecules using the acquired component for an assay.

In this case, the component for an assay may include the processing/analysis module including sub-modules for processing and analyzing the molecular diagnostic amplification data for the plurality of target nucleic acid molecules in the target presence/absence determining software as described above, and the parameter values referenced by the sub-modules.

Here, the sub-module predefined to be referenced during the processing and analyzing of the molecular diagnostic amplification data among the sub-modules processes and analyzes the molecular diagnostic amplification data for the plurality of target nucleic acid molecules by referencing the parameter value in response to the request of the target presence/absence determining software.

In an embodiment, the device 100 for generating a component for an assay described above may be implemented as a computing device.

The computing device may include one or more processors, a bus, a communication interface, a memory for loading a computer program executed by the processor, and a storage for storing the computer program. One of ordinary skill in the art to which the present embodiment pertains will understand that, in addition to these components, other general-purpose components may also be included.

The processor may control the overall operations of each component of the computing device. The processor may include at least one of a Central Processing Unit (CPU), a Microprocessor Unit (MPU), a Microcontroller Unit (MCU), a Graphics Processing Unit (GPU), or any type of processor well known in the technical field of the present embodiment. The processor may also perform computations for at least one application or program for executing the methods/operations according to various embodiments described herein. The computing device may include one or more processors.

The memory may store various data, commands, and/or information. The memory may load one or more programs from the storage in order to execute the methods/operations according to various embodiments described herein. An example of the memory may be RAM, but is not limited thereto.

The bus may provide communication functionality among the components of the computing device. The bus may be implemented in various forms, including an address bus, a data bus, and a control bus.

The communication interface may support wired or wireless internet communication of the computing device. The communication interface may also support various types of communication other than Internet communication. To this end, the communication interface may include a communication module well known in the technical field of the present embodiment.

The storage may non-transitorily store one or more computer programs. The storage may include non-volatile memory such as Read-Only Memory (ROM), Erasable Programmable ROM (EPROM), Electrically Erasable Programmable ROM (EEPROM), flash memory, a hard disk, a removable disk, or any type of non-transitory computer-readable storage medium well known in the technical field to which one embodiment of the present invention pertains.

The computer program may include one or more instructions implementing the methods/operations according to various embodiments described herein. When the computer program is loaded into memory, the processor may execute one or more instructions to perform the methods/operations according to various embodiments described herein.

In an embodiment, the computer program may include instructions for acquiring molecular diagnostic amplification data generated by using a molecular diagnostic assay for a plurality of target nucleic acid molecules, and instructions for generating the component for the assay based at least partially on the molecular diagnostic amplification data. Here, the component for the assay includes a processing/analysis module including sub-modules for processing and analyzing, in the target presence/absence determining software, the molecular diagnostic amplification data for the plurality of target nucleic acid molecules, and a parameter value referenced by the sub-modules, and wherein a sub-module predefined to be called for the processing and analyzing the molecular diagnostic amplification data among the sub-modules processes and analyzes the molecular diagnostic amplification data for the plurality of target nucleic acid molecules by referencing the parameter value in response to a request of the target presence/absence determining software.

In an embodiment, the computer program may include instructions for acquiring molecular diagnostic amplification data generated using a molecular diagnostic assay for a plurality of target nucleic acid molecules, instructions for generating the component for the assay based at least partially on the molecular diagnostic amplification data, instructions for distributing the component for the assay to a target presence/absence determining terminal on which the target presence/absence determining software is stored. Here, wherein the component for the assay includes a processing/analysis module including sub-modules for processing and analyzing the molecular diagnostic amplification data for the plurality of target nucleic acid molecules in the target presence/absence determining software, and a parameter value referenced by the sub-modules, and wherein a sub-module predefined to be called for the processing and analyzing the molecular diagnostic amplification data among the sub-modules processes and analyzes the molecular diagnostic amplification data for the plurality of target nucleic acid molecules by referencing the parameter value in response to a request of the target presence/absence determining software.

The methods according to the embodiments described above may be performed by executing a computer program implemented in computer-readable code. The computer program may be transmitted from a first computing device to a second computing device via a network such as the internet, and installed on the second computing device so that it can be used therein. The first and second computing devices include, but are not limited to, fixed computing devices such as server devices, physical servers in a server pool for cloud services, and desktop PCs.

The computer program may also be stored on a storage medium such as a DVD-ROM or a flash memory device.

Such a program may be stored on a non-transitory computer-readable storage medium to implement functionality in a specific manner. Further, unless otherwise specified, terms such as "include," "comprise," or "have" used herein shall be construed to imply the possibility of including other elements, rather than excluding them. All terms, including technical and scientific terms, shall be interpreted to have the same meanings as those generally understood by one of ordinary skill in the art to which this disclosure pertains, unless otherwise defined. Commonly used terms shall be interpreted in accordance with their context in the relevant technology and not in an overly idealized or excessively formal sense, unless explicitly defined herein.

The above description is merely exemplary description of the technical scope of the present disclosure, and it will be understood by those skilled in the art that various changes and modifications can be made without departing from original characteristics of the present disclosure. Therefore, the embodiments disclosed in the present disclosure are intended to explain, not to limit, the technical scope of the present disclosure, and the technical scope of the present disclosure is not limited by the embodiments. The protection scope of the present disclosure should be interpreted based on the following claims and it should be appreciated that all technical scopes included within a range equivalent thereto are included in the protection scope of the present disclosure.

While specific parts of the present disclosure have been described in detail above, it will be apparent to those skilled in the art that such specific descriptions are merely exemplary implementations and are not intended to limit the scope of the disclosure. Accordingly, the true scope of the present disclosure shall be defined by the appended claims and their equivalents.

## Claims

1. A method performed by a generation device for generating an assay component used in a target presence/absence-determining software displaying molecular diagnostic amplification data, the method comprising:
obtaining molecular diagnostic amplification data generated using a molecular diagnostic assay for a plurality of target nucleic acid molecules;
generating the assay component at least partially based on the molecular diagnostic amplification data;
wherein the assay component comprises:
a processing/analysis module including sub-modules for processing and analyzing the molecular diagnostic amplification data for the plurality of target nucleic acid molecules in the target presence/absence-determining software; and
a parameter value referenced by the sub-modules, and
wherein one or more of the sub-modules, which are predefined to be called during processing and analysis of the molecular diagnostic amplification data, process and analyze the molecular diagnostic amplification data for the plurality of target nucleic acid molecules by referencing the parameter value in response to a request from the target presence/absence-determining software.

2. A method for distributing an assay component performed by a generation device for the assay component used in a target presence/absence-determining software displaying molecular diagnostic amplification data, the method comprising:
obtaining molecular diagnostic amplification data generated using a molecular diagnostic assay for a plurality of target nucleic acid molecules;
generating the assay component at least partially based on the molecular diagnostic amplification data;
distributing the assay component to a terminal on which the target presence/absence-determining software is stored,
wherein the assay component comprises:
a processing/analysis module including sub-modules for processing and analyzing the molecular diagnostic amplification data for the plurality of target nucleic acid molecules in the target presence/absence-determining software; and
a parameter value referenced by the sub-modules, and
wherein one or more of the submodules, which are predefined to be referenced during processing and analysis of the molecular diagnostic amplification data, process and analyze the data for the plurality of target nucleic acid molecules by referencing the parameter value in response to a request from the target presence/absence-determining software.

3. The method of claim 1 or 2, wherein the processing/analysis module is a common module for at least two target nucleic acid molecules among the plurality of target nucleic acid molecules.

4. The method of claim 1 or 2, wherein the processing/analysis module is an individual module provided for each of the plurality of target nucleic acid molecules.

5. The method of claim 1 or 2, wherein the processing/analysis module includes a common module provided for some of the plurality of target nucleic acid molecules and an individual module provided for some of the plurality of target nucleic acid molecules.

6. The method of claim 3, wherein the common module includes common sub-modules that are commonly used for the at least two target nucleic acid molecules among the plurality of target nucleic acid molecules.

7. The method of claim 3, wherein the individual module includes individual sub-modules that are individually used for each of the plurality of target nucleic acid molecules.

8. The method of claim 1 or 2, wherein, when the assay component includes at least two processing/analysis modules, the at least two processing/analysis modules includes sub-modules that are at least partially identical to each other.

9. The method of claim 1 or 2, wherein, when an additional assay component is generated for a different set of a plurality of target nucleic acid molecules from the plurality of target nucleic acid molecules, the additional assay component includes a processing/analysis module for processing and analyzing molecular diagnostic amplification data for the different set of the plurality of target nucleic acid molecules.

10. The method of claim 1 or 2, wherein the parameter value is predefined to be differentially referenced for each of the plurality of target nucleic acid molecules when the processing/analysis module performs the processing and analyzing.

11. The method of claim 1 or 2, wherein the sub-modules include an algorithm that analyzes or mathematically processes a data set acquired during an amplification of the plurality of target nucleic acid molecules.

12. The method of claim 1 or 2, wherein the sub-modules include an algorithm that performs an operation of outputting a cycle number when a data set acquired during an amplification of the plurality of target nucleic acid molecules reaches a threshold value and an operation of determining presence/absence of the target nucleic acid molecules based at least partially on the cycle number output.

13. The method of claim 1 or 2, wherein the sub-modules include an algorithm that performs one of the following operations:
(i) analyzing signals detected at a relatively high detection temperature and a relatively low detection temperature during an amplification of the plurality of target nucleic acid molecules,
(ii) normalizing a plurality of data set acquired during the amplification of the plurality of target nucleic acid molecules,
(iii) determining the presence/absence of the target nucleic acid molecules using a parameter representing a maximum derivative value of a sigmoid fitting curve for the data set acquired during the amplification of the plurality of target nucleic acid molecules,
(iv) evaluating a shape of the sigmoid fitting curve using a value representing a profile of the data set acquired during the amplification of the plurality of target nucleic acid molecules,
(v) performing a regression analysis on the data set acquired during the amplification of the plurality of target nucleic acid molecules, and
(vi) performing a regression model analysis on a portion of the data set acquired through a signal generation reaction for the target nucleic acid molecules.

14. The method of claim 1 or 2, wherein the parameter value is used for signal processing of the target presence/absence-determining software or serves as a criterion for a determination made by the signal processing.

15. The method of claim 1 or 2, wherein the parameter value is (i) a parameter value defining a criterion for determining whether a value of a data set acquired during an amplification of the plurality of target nucleic acid molecules exceeds a threshold value, or
(ii) a parameter value defining a criterion for determining the presence/absence of the target nucleic acid molecules using a cycle number at which the value of the data set acquired during the amplification of the plurality of target nucleic acid molecules reaches the threshold value.

16. The method of claim 1 or 2, wherein the parameter value is any one of
(i) a parameter value indicating a relationship between a magnitude of a signal detected at a relatively high detection temperature and a magnitude of a signal detected at a relatively low detection temperature in a data set acquired during an amplification of the plurality of target nucleic acid molecules;
(ii) a parameter value defining a normalization coefficient for normalizing a plurality of data sets acquired during the amplification of the plurality of target nucleic acid molecules;
(iii) a parameter value defining a criterion for determining the absence of the target nucleic acid molecules using a maximum derivative value of a sigmoid fitting curve applied to the plurality of data sets acquired during the amplification of the plurality of target nucleic acid molecules;
(iv) a parameter value defining a criterion for determining the presence of the target nucleic acid molecules using a parameter representing the maximum derivative value of the sigmoid fitting curve applied to the plurality of data sets acquired during the amplification of the plurality of target nucleic acid molecules;
(v) a parameter value defining a criterion for determining a shape of the sigmoid fitting curve applied to the plurality of data sets acquired during the amplification of the plurality of target nucleic acid molecules;
(vi) a parameter value defining a start cycle required for performing a regression analysis on the plurality of data sets acquired during the amplification of the plurality of target nucleic acid molecules;
(vii) a parameter value representing a minimum cycle of a final cycle required for performing the regression analysis on the plurality of data sets acquired during the amplification of the plurality of target nucleic acid molecules;
(viii) a parameter value defining a criterion for determining the absence of the target nucleic acid molecules using fitting accuracy acquired by applying a regression model applied to a portion of the plurality of data sets acquired during the amplification of the plurality of target nucleic acid molecules;
(ix) a parameter value defining a criterion for determining the absence of the target nucleic acid molecules using fitting accuracy acquired by applying regression model analysis to all of the plurality of data sets acquired during the amplification of the plurality of target nucleic acid molecules; and
(x) a parameter value defining a criterion for determining the absence of the target nucleic acid molecules using a mathematical processing of a maximum value and a minimum value or a mathematical processing of a value at a final cycle and a minimum value of the plurality of data sets acquired during the amplification of the plurality of target nucleic acid molecules.

17. The method of claim 1 or 2, wherein the assay component further includes assay information describing the plurality of target nucleic acid molecules for the molecular diagnostic assay.

18. The method of claim 1 or 2, wherein the assay component includes assay information that matches the sub-modules to parameter values referenced by the sub-modules for each target nucleic acid molecule.

19. The method of claim 1 or 2, wherein, when the processing/analysis module includes two or more sub-modules, the assay component further includes assay information in which an execution order of the sub-modules is predefined.

20. The method of claim 20 wherein the execution order is predefined for each of the plurality of target nucleic acid molecules.

21. The method of claim 1 or 2, wherein the processing/analysis module and the parameter value are designed through a process of optimizing performance of the molecular diagnostic assay.

22. The method of claim 22 wherein the process of optimizing the performance of the molecular diagnostic assay includes repeatedly determining a parameter value referenced by pre-designated sub-modules until performance of the molecular diagnostic assay satisfies a predetermined criterion, and
when the performance of the molecular diagnostic assay does not satisfy the predetermined criterion after the repetition is performed, a redesign of the pre-designated sub-modules is further performed.

23. The method of claim 23, wherein the process of redesigning the pre-designated sub-modules includes at least one of modifying one or more sub-modules included in the pre-designated sub-modules, adding to the assay component sub-modules not included in the pre-designated sub-modules, and deleting at least one of the sub-modules or changing an execution order of the sub-modules when the pre-designated sub-modules include two or more sub-modules.

24. The method of claim 2, wherein the assay component is distributed to the terminal with the target presence/absence-determining software in order to address an issue of noise or abnormal signal removal in the molecular diagnostic amplification data that cannot be resolved by a pre-stored assay component existing in the terminal.

25. The method of claim 2, wherein the assay component is distributed to the terminal with the target presence/absence-determining software in order to overwrite a pre-stored assay component existing in the terminal.

26. The method of claim 2, wherein the assay component is distributed to the terminal with the target presence/absence-determining software in order to cause the terminal to include the assay component together with a pre-stored assay component existing in the terminal.

27. The method of claim 2, wherein the assay component is installed in the terminal with the target presence/absence-determining software by a user who is authorized to install the assay component.

28. The method of claim 2, wherein the assay component is deleted from the terminal with the target presence/absence-determining software by a user who is authorized to delete the assay component.

29. The method of claim 1 or 2, wherein the molecular diagnostic amplification data is acquired from a result of a nucleic acid polymerase chain-reaction (PCR) targeting the plurality of target nucleic acid molecules.

30. A method performed by a target presence/absence-determining terminal for determining target presence/absence using an assay component used in used in target presence/absence-determining software displaying molecular diagnostic amplification data, the method comprising:
obtaining an assay component generated at least partially based on molecular diagnostic amplification data generated using a molecular diagnostic assay for a plurality of target nucleic acid molecules; and
processing/analysis the molecular diagnostic amplification data for the plurality of target nucleic acid molecules using the assay component,
wherein the assay component comprises:
a processing/analysis module including sub-modules for processing and analyzing the molecular diagnostic amplification data for the plurality of target nucleic acid molecules in the target presence/absence-determining software; and
a parameter value referenced by the sub-modules, and
wherein one or more of the submodules, which are predefined to be referenced during processing and analysis of the molecular diagnostic amplification data, process and analyze the data for the plurality of target nucleic acid molecules by referencing the parameter value in response to a request from the target presence/absence-determing software.

31. A computer-readable recording medium storing a computer program for generating an assay component used in target presence/absence-determining software that displays molecular diagnostic amplification data, the computer program comprising:
wherein the computer program comprises:
instructions for obtaining molecular diagnostic amplification data generated using a molecular diagnostic assay for a plurality of target nucleic acid molecules; and
instructions for generating the assay component at least partially based on the molecular diagnostic amplification data,
wherein the assay component comprises:
a processing/analysis module including sub-modules for processing and analyzing the molecular diagnostic amplification data for the plurality of target nucleic acid molecules in the target presence/absence-determining software; and
a parameter value referenced by the sub-modules, and
wherein one or more of the submodules, which are predefined to be referenced during processing and analysis of the molecular diagnostic amplification data, process and analyze the data for the plurality of target nucleic acid molecules by referencing the parameter value in response to a request from the target presence/absence-determing software.

32. A computer-readable recording medium storing a computer program for distributing an assay component used in target presence/absence-determining software that displays molecular diagnostic amplification data, the computer program comprising:
wherein the computer program comprises:
instructions for obtaining molecular diagnostic amplification data generated using a molecular diagnostic assay for a plurality of target nucleic acid molecules; and
instructions for generating the assay component at least partially based on the molecular diagnostic amplification data,
instructions for distributing the assay component to a terminal on which the target presence/absence-determining software is stored,
wherein the assay component comprises:
a processing/analysis module including sub-modules for processing and analyzing the molecular diagnostic amplification data for the plurality of target nucleic acid molecules in the target presence/absence-determining software; and
a parameter value referenced by the sub-modules, and
wherein one or more of the submodules, which are predefined to be referenced during processing and analysis of the molecular diagnostic amplification data, process and analyze the data for the plurality of target nucleic acid molecules by referencing the parameter value in response to a request from the target presence/absence-determing software.

33. A device for generating an assay component used in target presence/absence-determining software displaying molecular diagnostic amplification data, the device comprising:
a processor;
a memory; and
a computer program loaded in the memory and executed by the processor,
wherein the computer program comprises:
instructions for obtaining molecular diagnostic amplification data generated using a molecular diagnostic assay for a plurality of target nucleic acid molecules; and
instructions for generating the assay component at least partially based on the molecular diagnostic amplification data,
instructions for distributing the assay component to a terminal on which the target presence/absence-determining software is stored,
wherein the assay component comprises:
a processing/analysis module including sub-modules for processing and analyzing the molecular diagnostic amplification data for the plurality of target nucleic acid molecules in the target presence/absence-determining software; and
a parameter value referenced by the sub-modules, and
wherein one or more of the submodules, which are predefined to be referenced during processing and analysis of the molecular diagnostic amplification data, process and analyze the data for the plurality of target nucleic acid molecules by referencing the parameter value in response to a request from the target presence/absence-determing software.

34. A device for generating an assay component used in target presence/absence-determining software displaying molecular diagnostic amplification data, the device comprising:
a processor;
a memory; and
a computer program loaded in the memory and executed by the processor,
wherein the computer program comprises:
instructions for obtaining molecular diagnostic amplification data generated using a molecular diagnostic assay for a plurality of target nucleic acid molecules; and
instructions for generating the assay component at least partially based on the molecular diagnostic amplification data,
instructions for distributing the assay component to a terminal on which the target presence/absence-determining software is stored,
wherein the assay component comprises:
a processing/analysis module including sub-modules for processing and analyzing the molecular diagnostic amplification data for the plurality of target nucleic acid molecules in the target presence/absence-determining software; and
a parameter value referenced by the sub-modules, and
wherein one or more of the submodules, which are predefined to be referenced during processing and analysis of the molecular diagnostic amplification data, process and analyze the data for the plurality of target nucleic acid molecules by referencing the parameter value in response to a request from the target presence/absence-determing software.
